# EUROPÄISCHE PATENTSCHRIFT

(11) **EP 4 197 432 B1**
(45) Veröffentlichungstag und Bekanntmachung des Hinweises auf die Patenterteilung: **28.01.2026**
(21) Anmeldenummer: 22210840.9
(22) Anmeldetag: 01.12.2022
(51) Int. Cl.: A61B 5/00, H01F 38/14, H04B 5/00, H04L 25/02, G08C 17/02

(54) **MEDIZINISCHE VORRICHTUNG MIT GALVANISCHER TRENNEINRICHTUNG**
MEDICAL DEVICE WITH GALVANIC ISOLATING DEVICE
DISPOSITIF MÉDICAL DOTÉ D'UN DISPOSITIF DE SÉPARATION GALVANIQUE

(30) Priorität: 14.12.2021 DE 202021106775 U
(43) Veröffentlichungstag der Anmeldung: 21.06.2023
(73) Patentinhaber: Richard Wolf GmbH, 75438 Knittlingen (DE)
(72) Erfinder: Neurohr, Dipl.-Ing. Bernd, 75038 Oberderdingen/Grossvillars (DE); Send, Robert, 76137 Karlsruhe (DE)
(74) Vertreter: Patentanwälte Hemmer Lindfeld Frese Partnerschaft mbB

(56) Entgegenhaltungen:
- WO-A1-2017/036935
- JP-A- 2020 110 307
- US-A1- 2019 372 276

## Beschreibung

Die Erfindung betrifft eine medizinische Vorrichtung mit einer mit einem zu behandelnden Patienten in Berührung bringbare Anwendungseinrichtung und eine mit der Anwendungseinrichtung verbindbare galvanische Trenneinrichtung mit den im Oberbegriff des Anspruchs 1 angegebenen Merkmalen.

Bei medizinischen Vorrichtungen, die mit potenziell gefährlichen Spannungen ("PGS") arbeiten, muss sichergestellt werden, dass keine direkte, elektrisch leitende Verbindung zwischen einer Spannungsquelle und dem Patienten existiert. Bei endoskopischen Kameras ist beispielsweise vorgesehen, dass zwischen einem Netzteil und einer endoskopischen Optik mindestens ein galvanisches Trennelement vorgesehen ist, das eine direkte, elektrisch leitende Verbindung verhindert. Unter anderem die Norm IEC60601-1 macht Vorgaben zu Luft- und Kriechstrecken, Spannungsfestigkeit und möglichen Ableitströmen durch einen Patienten. Die Umsetzung einer elektromagnetischen Abschirmung bzw. Verträglichkeit gegen Einstrahlung und Aussendung von elektromagnetischen Störungen ist bei Elektrogeräten mit galvanischer Trennung besonders anspruchsvoll und mit höheren Herstell- und Entwicklungskosten verbunden. Eine Herausforderung ist unter anderem, dass nicht alle elektronischen Bauteile auf dieselbe, durch das Leitungsnetz vorgegebene Masse gelegt werden können, welche üblicherweise für die Abschirmung genutzt wird. So könnte bereits die Anbindung eines Niederspannungs-Geräts, beispielsweise eine handelsübliche Computermaus, zu unerwünschten Störstrahlungen führen.

Zusätzlich zu einer galvanischen Trennfunktion besteht oftmals das Erfordernis, große Datenmengen zu übertragen. Zwar existieren Bauteile, welche Daten über galvanische Trennstrecken übertragen können, diese sind jedoch kostenintensiv oder in ihren realisierbaren Datenraten deutlich begrenzt. In der US 2019/0372276 A1 ist eine galvanische Trennung innerhalb einer Kabelsteckverbindung eines Patientenüberwachungssystems beschrieben.

Vor diesem Hintergrund liegt der Erfindung die Aufgabe zugrunde, eine medizinische Vorrichtung vorzuschlagen, bei der eine einfach zu realisierende und dennoch wirkungsvolle galvanische Trennung ermöglicht wird, die hohe Datenraten erlaubt, kostengünstig herstellbar ist und zudem einfach überprüfbar ist.

Diese Aufgabe wird gemäß der Erfindung durch eine medizinische Vorrichtung mit den in Anspruch 1 angegebenen Merkmalen gelöst. Vorteilhafte Ausgestaltungen der Erfindung sind in den Unteransprüchen, der nachfolgenden Beschreibung und den Zeichnungen angegeben.

Die erfindungsgemäße medizinische Vorrichtung weist eine mit einem zu behandelnden Patienten in Berührung bringbare Anwendungseinrichtung, und eine galvanische Trenneinrichtung, die mit der Anwendungseinrichtung verbindbar ist, auf, wobei die Trenneinrichtung mindestens einen Anwendungsanschluss zum Verbinden mit der Anwendungseinrichtung und mindestens einen Versorgungsanschluss zum Verbinden mit einer Einrichtung aufweist, wobei die Trenneinrichtung dazu ausgebildet ist, den Anwendungsanschluss von dem Versorgungsanschluss galvanisch zu trennen. Erfindungsgemäß ist vorgesehen, dass die Trenneinrichtung mindestens eine mit dem Anwendungsanschluss verbundene erste Funkeinheit mit einer ersten Antenne und mindestens eine mit dem Versorgungsanschluss verbundene zweite Funkeinheit mit einer zweiten Antenne aufweist, wobei die jeweilige erste Antenne und die jeweilige zweite Antenne auf einem Träger mit bloßem Auge erkennbar voneinander beabstandet und zueinander gerichtet fixiert sind, und wobei die mindestens eine erste Funkeinheit und die mindestens eine zweite Funkeinheit dazu ausgebildet sind, Signale bzw. Daten zwischen dem Anwendungsanschluss und dem Versorgungsanschluss zu übertragen.

Das Merkmal, dass die jeweilige erste Antenne und die jeweilige zweite Antenne auf einem Träger "mit bloßem Auge" erkennbar voneinander beabstandet sind, kann hier beispielsweise bedeuten, dass für die Erkennung des Abstands zwischen den Antennen diese nicht zur mikroskopischen Untersuchung ausgebaut werden müssen. Typischerweise trifft dies bereits für Abstände oberhalb von 0,5 mm zu. Vorzugsweise beträgt der Abstand zwischen den Antennen jedoch mindestens 0,5 cm, was bei normaler Sehstärke mit bloßem Auge und ohne jegliches optisches Hilfsmittel erkennbar ist. Vorzugsweise ist der Abstand frei einsehbar angeordnet und nicht verdeckt, also bei der Prüfung der Vorrichtung ohne vorherigen Teileausbau, abgesehen von einer etwaigen Öffnung eines Gehäuses der Vorrichtung, direkt sichtbar. Erfindungsgemäß sind die jeweilige erste Antenne und die jeweilige zweite Antenne zueinander gerichtet fixiert. Dies ist beispielsweise so zu verstehen, dass die erste Antenne eine erste Hauptfunkrichtung zum Senden und/oder Empfangen von Funksignalen definiert und die zweite Antenne eine zweite Hauptfunkrichtung zum Senden und/oder Empfangen von Funksignalen definiert, wobei die erste Hauptfunkrichtung zur zweiten Hauptfunkrichtung koaxial entgegengesetzt ausgerichtet ist.

Die Anwendungseinrichtung kann auf vielfältige, unterschiedliche Arten ausgeführt sein. Beispielsweise könnte sie eine Endoskopvorrichtung und/oder einen Sensor, Elektroden, Bildaufnehmer, Ultraschallwander und dergleichen aufweisen. Die Anwendungseinrichtung kann aufgrund ihrer jeweiligen Konzeption das Bereitstellen von Signalen bzw. Daten ermöglichen. Es sind beliebige andere Einrichtungen denkbar, die direkt mit einem Patienten in Berührung bringbar sind und in denen eine galvanische Trenneinrichtung integrierbar ist. Andererseits könnte die galvanische Trenneinrichtung auch in eine mit einer Anwendungseinrichtung verbindbare Komponente, beispielsweise eine Verarbeitungseinheit, integriert werden. Beispielsweise könnte die galvanische Trenneinrichtung auch eine Daten- oder Signalübertragung zwischen direkt benachbarten Geräten ermöglichen. Die Geräte könnten metallische Gehäuse mit einer Aussparung aufweisen, durch die eine Signal- bzw. Datenübertragung erfolgt. Bei korrekter Ausrichtung der Geräte zueinander könnte folglich eine Datenübertragung realisiert werden. Eine Ausrichtung könnte durch Führungen, Vertiefungen oder Magnete begünstigt werden. Eine galvanische Trenneinrichtung könnte etwa einseitig in einen Gehäusefuß integriert sein.

Die galvanische Trenneinrichtung dient der Übertragung von Signalen bzw. Daten zwischen der Anwendungseinrichtung und einer Einrichtung, die mit der Anwendungseinrichtung zu verbinden ist. Die Trenneinrichtung bildet folglich eine Schnittstelle aus, die einen elektrisch sicheren Betrieb der Anwendungseinrichtung am Patienten ermöglicht.

Eine Besonderheit liegt in der Verwendung der mindestens einen ersten Funkeinheit und der mindestens einen zweiten Funkeinheit, welche jeweils mit einer Antenne verbunden und auf dem Träger mit bloßem Auge erkennbar voneinander beabstandet sind. Eine mechanische und elektrische Trennung zwischen zwei galvanisch voneinander getrennten Bereichen ist demnach ohne aufwändige Prüfung vollkommen offensichtlich und daher leicht überprüfbar. Eine Zulassung für eine medizinische Vorrichtung kann folglich ohne eine technisch aufwändige Prüfung wie bei vorangehend erwähnten Vorrichtungen aus dem Stand der Technik erfolgen.

Die funkbasierte Verbindung kann über einen Kurzstreckenfunk mit geringer Funkleistung erfolgen. Es ist vorstellbar, eine Funkleistung in einem Bereich von 0,01 µW bis 300 mW und insbesondere von 0,02 µW bis 193 mW zu realisieren. Entsprechende Baugruppen sind für Datenübertragungen bekannt. Der Kurzstreckenfunk kann etwa im GHz-Bereich arbeiten, etwa im 60 GHz V-Band mit Funkfrequenzen im Bereich 57 GHz und 64 GHz. Dies hat den Vorteil, dass die Aussendung leicht durch metallische Schichten abgeschirmt werden können. Die galvanische Trenneinrichtung sendet folglich selbst keine störende Strahlung aus, wenn sie von einem leitfähigen Gehäuse umgeben ist.

Ein Zwischenraum zwischen der ersten Antenne und der zweiten Antenne fungiert als Isolations- bzw. Übertragungsstrecke. Der Zwischenraum kann derart ausgelegt werden, dass mindestens übliche Normen zu Luft- und Kriechstrecken erfüllt werden. Er kann lediglich mit Luft gefüllt sein, allerdings auch bestimmte Werkstoffe enthalten, etwa Kunststoffe und insbesondere einen Platinenwerkstoff.

Es ist bevorzugt, wenn an dem Versorgungsanschluss zusätzlich ein besonders bevorzugt zweiphasiger Schalter vorgesehen ist und/oder eine bevorzugt zweiphasige Gerätesicherung.

Die Trenneinrichtung könnte zwei erste Funkeinheiten und zwei zweite Funkeinheiten aufweisen, wobei die ersten Funkeinheiten und die zweiten Funkeinheiten paarweise einander zugewandt sind, und wobei eines der Paare zum Übertragen von Signalen bzw. Daten von der ersten Funkeinheit an die zweite Funkeinheit und das andere der Paare zum Übertragen von Signalen bzw. Daten von der zweiten Funkeinheit an die erste Funkeinheit ausgebildet ist. Folglich kann die Trenneinrichtung eine Duplex-Übertragung realisieren, bei der Signale bzw. Daten in zwei Richtungen übertragbar sind. Hierbei können Signale bzw. Daten in beide Richtungen gleichzeitig übertragen werden.

Die mindestens eine erste Funkeinheit und die mindestens eine zweite Funkeinheit könnte jeweils als Sende- und Empfangseinheit ausgeführt sein. Die Signal- bzw. Datenübertragung kann demnach grundsätzlich in beiden Richtungen erfolgen. Liegt lediglich ein einzelnes Paar bestehend aus einer ersten Funkeinheit und einer zweiten Funkeinheit vor, kann eine Semi-Duplex-Verbindung realisiert sein.

Eine zwischen der ersten Antenne und der zweiten Antenne vorliegende Funkstrecke könnte eine Ausdehnung von 0,5 bis 60 cm und besonders bevorzugt von 1 bis 40 cm aufweisen. Die Ausdehnung der Funkstrecke über eine Distanz von mindestens 0,5 cm ist mit bloßem Auge sehr leicht zu erkennen. Dies vereinfacht deutlich die Prüfung der Trenneinrichtung. Eine hier mit maximal 60 cm angegebene Ausdehnung kann die notwendige Funkleistung deutlich begrenzen. Insbesondere bei starkem Richtcharakter der Antennen kann damit sowohl eine wirkungsvolle galvanische Trennung, als auch eine Minimierung der Funkleistung erreicht werden.

Der Träger könnte als eine einzelne Platine ausgeführt sein, auf der die mindestens eine erste Funkeinheit und die mindestens eine zweite Funkeinheit in einem vorbestimmten Abstand zueinander montiert sind. Dies ist mechanisch besonders einfach, denn die erste Funkeinheit und die zweite Funkeinheit bzw. deren Antennen müssen lediglich an vorgesehenen Positionen auf der Platine befestigt werden, um die gewünschte Funkverbindung herzustellen. Die Form des Trägers ist zudem sehr leicht an die medizinische Vorrichtung anpassbar und könnte insbesondere in die Anwendungseinrichtung integriert werden.

Die Platine könnte eine Aussparung zwischen der ersten Funkeinheit und der zweiten Funkeinheit aufweisen. Die Aussparung könnte dazu dienen, eine noch weiter verbesserte optische Überprüfung der galvanischen Trennung zu erreichen. Die Aussparung könnte insbesondere einen rechteckigen Ausschnitt aufweisen, der mit bloßem Auge erkennbar eine Trennung zwischen der ersten Funkeinheit und der zweiten Funkeinheit darstellt. Die Antennen sind dabei bevorzugt randseitig an einander gegenüberliegenden Kanten der Aussparung angeordnet und bilden folglich ihre Funkstrecke über der Aussparung aus.

Der Träger könnte eine erste Platine und eine zweite Platine aufweisen, die in einem vorbestimmten Abstand zueinander angeordnet und mechanisch miteinander verbunden sind, wobei die mindestens eine erste Funkeinheit auf der ersten Platine angeordnet ist und die mindestens eine zweite Funkeinheit auf der zweiten Platine angeordnet ist. Insbesondere bei größeren bzw. längeren medizinischen Vorrichtungen kann es sinnvoll sein, statt einer einzelnen Platine zwei voneinander getrennte Platinen zu verwenden, die mechanisch in einem vorbestimmten Abstand zueinander angeordnet und befestigt sind. Es kann sich etwa anbieten, eine Platine an einem distalen Ende der Anwendungseinrichtung anzuordnen und die andere Platine in Richtung eines proximalen Endes zu versetzen. Die Anwendungseinrichtung kann dabei ein Gehäuse umfassen, das die beiden Platinen umschließt und im Inneren Befestigungspunkte aufweist, die eine Befestigung der Platinen ermöglicht.

Die erste Antenne und die zweite Antenne könnten auf den Träger gedruckt sein. Das Drucken könnte durch Aufbringen und Fixieren leitfähiger Partikel, durch Ätzen oder ein anderes Herstellverfahren erreicht werden. Dadurch ergibt sich eine besonders einfach herstellbare Trenneinrichtung, die kostengünstig und räumlich kompakt ist. Dies erlaubt insbesondere eine Realisierung in Anwendungseinrichtungen mit kleinem Querschnitt.

Weiterhin könnten die erste Antenne und die zweite Antenne jeweils eine Hornstrukturzum Herstellen einer gerichteten Funkverbindung aufweisen. Die Hornstruktur unterstützt den starken Richtcharakter der Antennen. Die Datenraten können hierbei gesteigert werden. Es ist vorstellbar, Multi-Link-Anwendungen zu realisieren, bei der zwei unterschiedliche Hornstrukturen mit vertikaler und horizontaler Polarisation eingesetzt werden, um die Datenraten noch weiter zu steigern. Neben Hornstrukturen könnten auch Hornantennen eingesetzt werden, die eine integrierte Antenne aufweisen.

Die Trenneinrichtung könnte eine mit dem Anwendungsanschluss verbundene erste Kopplungseinheit und eine mit dem Versorgungsanschluss verbundene zweite Kopplungseinheit aufweisen, die dazu ausgebildet sind, induktiv und/oder kapazitiv elektrische Leistung von dem Versorgungsanschluss an den Anwendungsanschluss zu übertragen. Die beiden Kopplungseinheiten erlauben demzufolge, eine elektrische Leistung drahtlos zu übertragen. Dadurch könnte eine distal angeordnete Beleuchtungseinrichtung, eine Sensoreinrichtung oder jegliche andere Arten von Verbrauchern mit elektrischer Leistung versorgt werden, ohne eine direkte Verbindung zwischen dem Versorgungsanschluss und der Anwendungseinrichtung zu erfordern. Es bietet sich dabei besonders an, eine induktive Übertragung zu realisieren, die zwei miteinander koppelbare Spulen umfasst. Die beiden Spulen können in Fluchtung gebracht werden, sodass ein an dem Versorgungsanschluss angelegte Spannung in eine entsprechende Spule induziert wird, die mit der Anwendungseinrichtung verbunden ist.

Die Trenneinrichtung könnte von einem Gehäuse umschlossen sein. Das Gehäuse könnte ein separates Gehäuse sein, welches innerhalb der Anwendungseinrichtung platzierbar ist. Es ist auch denkbar, die Trenneinrichtung so von einem Gehäuse zu umschließen, dass die Funkstrecke zwischen den Antennen von außen sichtbar ist. Es ist etwa denkbar, das Gehäuse mit einem transparenten, nichtleitenden Kunststoff zu füllen. Das Gehäuse könnte auch ein der Anwendungseinrichtung zugeordnetes Gehäuse sein, in dem lediglich die Trenneinrichtung angeordnet ist und nicht unbedingt ein eigenes, separates Gehäuse umfasst.

Ein die Funkstrecke ganz oder teilweise umgebendes Gehäuse könnte metallisch leitfähig sein, mit Leitfähigkeiten größer als 0,0004 S/m, um eine elektromagnetische Abschirmung zu erreichen. Das Gehäuse könnte ferner ein Kunststoffgehäuse sein. Die bevorzugte Wandstärke kann 0,5 mm übersteigen und beispielsweise bis zu etwa 3 mm betragen. Es ist besonders bevorzugt, eine chemische Beständigkeit einer Gehäuseoberfläche gegenüber Alkoholen, Aldehyden, Wasser und Tensiden vorzusehen, um eine gute Reinigbarkeit zu erreichen.

Es ist vorteilhaft, wenn ein Verhältnis v=d/L zwischen einer Distanz d zwischen der mindestens einen ersten Funkeinheit und der mindestens einen zweiten Funkeinheit sowie einer Wellenlänge L der Signale in einem Bereich von 1,73 bis 127 liegt. Dadurch kann eine hervorragende Signal- bzw. Datenübertragung realisiert werden.

Ein Bereich zwischen der mindestens einen ersten Funkeinheit und der mindestens einen zweiten Funkeinheit könnte zumindest teilweise mit einem Material ausgefüllt sein, das eine Permittivität von 1 bis 23 F/m und eine Leitfähigkeit von 0 bis 0,01 S/m aufweist. Besonders bevorzugt könnte dies Luft, ein Kunststoff oder Platinenmaterial sein.

Der Bereich kann weiterhin bevorzugt mit Medien gefüllt sein, deren Absorptionskoeffizient I/I₀ für Licht im Bereich 400nm-750nm entlang der Funkstrecke den Wert 0,7 nicht übersteigt.

Die Trenneinrichtung ist bevorzugt derart ausgebildet, dass die Dämpfung der Sendeleistung bei Raumtemperatur und Normaldruck zwischen 0,01 und 10 dB/km beträgt.

Die Erfindung ist nachfolgend anhand von in den Zeichnungen dargestellten Ausführungsbeispiels näher erläutert. Es zeigen:
- Fig. 1: eine schematische Übersicht über die medizinische Vorrichtung,
- Fig. 2 bis 6: jeweils eine schematische Darstellung einer galvanischen Trenneinrichtung,
- Fig. 7: eine schematische Darstellung einer Anwendungseinrichtung.

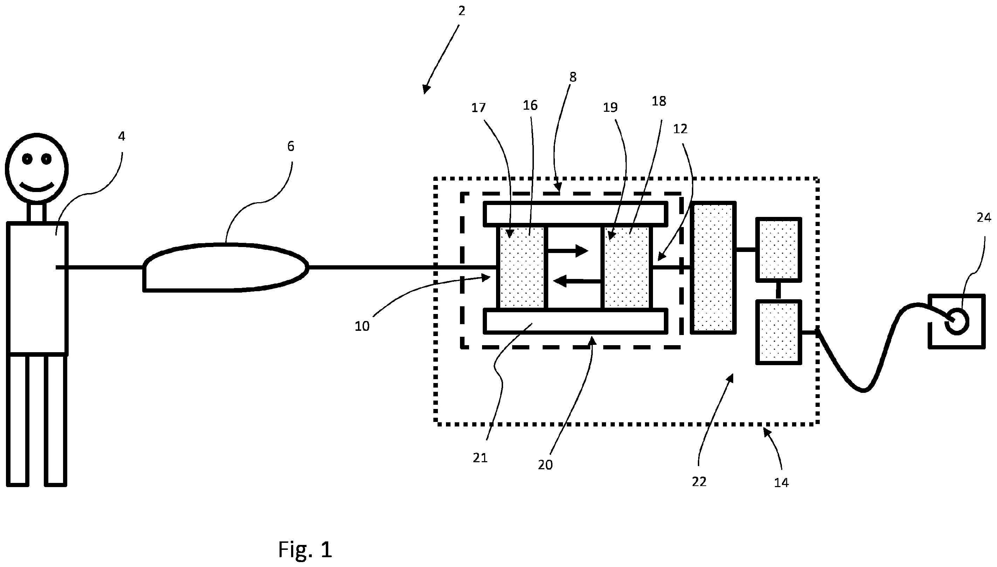

Fig. 1 zeigt eine medizinische Vorrichtung 2 zur Behandlung eines Patienten 4. Die medizinische Vorrichtung 2 weist eine Anwendungseinrichtung 6 auf, die mit dem Patienten 4 in Berührung bringbar ist. Sehr schematisch wird eine galvanische Trenneinrichtung 8 dargestellt, die mit der Anwendungseinrichtung 6 verbindbar ist. Die Trenneinrichtung 8 weist dabei einen Anwendungsanschluss 10 zum Verbinden mit der Anwendungseinrichtung 6 sowie einen Versorgungsanschluss 12 zum Verbinden mit einer Einrichtung 14 auf. Die Trenneinrichtung 8 ist dabei ausgebildet, den Anwendungsanschluss 10 von dem Versorgungsanschluss 12 galvanisch zu trennen. Hier ist die Trenneinrichtung 8 als Teil der Einrichtung 14 ausgeführt. Es ist jedoch auch denkbar, dass die Trenneinrichtung 8 Teil der Anwendungseinrichtung 6 ist.

Die Trenneinrichtung 8 weist eine mit dem Anwendungsanschluss 10 verbundene erste Funkeinheit 16 sowie eine mit dem Versorgungsanschluss 12 verbundene zweite Funkeinheit 18 auf. Die erste Funkeinheit 16 weist eine erste Antenne 17 auf, während die zweite Funkeinheit 18 eine zweite Antenne 19 umfasst. Die Trenneinrichtung 8 ist hier lediglich schematisch dargestellt, sodass in dieser Darstellung keine genaue Antennenstruktur erkennbar ist. Dies wird in weiter nachfolgenden Figuren detaillierter dargestellt.

Die erste Antenne 17 und die zweite Antenne 19 sind auf einem Träger 20 mit bloßem Auge erkennbar voneinander beabstandet angeordnet und zueinander gerichtet. Dadurch sind die erste Funkeinheit 16 und die zweite Funkeinheit 18 dazu ausgebildet, Signale bzw. Daten zwischen dem Anwendungsanschluss 10 und dem Versorgungsanschluss 12 zu übertragen. Es liegt keine direkte elektrische Verbindung zwischen dem Anwendungsanschluss 10 und dem Versorgungsanschluss 12 vor.

An dem Versorgungsanschluss 12 ist beispielhaft eine Anordnung von Platinen 22 vorgesehen, die mit einer Spannungsquelle 24 verbunden sind und den Versorgungsanschluss 12 mit einer entsprechenden Spannung bzw. elektrischen Leistung versorgen. Der Patient 4 erhält durch die galvanische Trenneinrichtung 8 jedoch keinen direkten Kontakt mit der Spannungsquelle 24.

Die erste Funkeinheit 16 und die zweite Funkeinheit 18 sind jeweils als eine Platine ausgeführt, die mittels mechanischer Fixierungen 21 miteinander verbunden sind. Die Platinen und die Fixierungen 21 bilden folglich den Träger 20 aus.

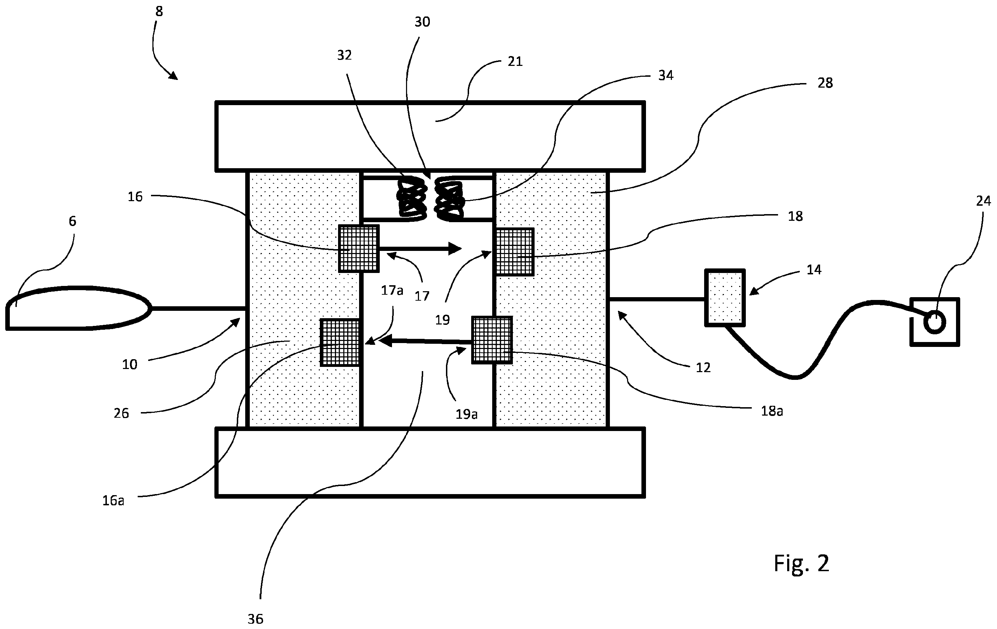

Fig. 2 zeigt ein Ausführungsbeispiel der galvanischen Trenneinrichtung 8 in einer etwas detaillierteren Darstellung. Hier ist eine erste Platine 26 mit darauf angeordneter erster Funkeinheit 16 dargestellt. Die erste Antenne 17 ist in der Zeichnungsebene nach rechts ausgerichtet und zeigt dort auf die zweite Antenne 19 der zweiten Funkeinheit 18, die wiederum auf einer zweiten Platine 28 angeordnet ist. Eine weitere erste Funkeinheit 16a ist auf der ersten Platine 26 angeordnet und weist eine weitere erste Antenne 17a auf. Auf diese ist eine zweite Antenne 19a gerichtet, die zu einer weiteren zweiten Funkeinheit 18a zugeordnet ist. Durch diese Anordnung kann auch bei Verwendung zweier unidirektionaler erster Funkeinheiten 16 und 16a sowie zweier unidirektionaler zweiter Funkeinheiten 18 und 18a eine bidirektionale Funkverbindung zwischen dem Versorgungsanschluss 12 und dem Anwendungsanschluss 10 realisiert werden.

Zusätzlich dazu ist eine Kupplungseinrichtung 30 vorgesehen, die eine erste Kopplungseinheit 32 und eine zweite Kopplungseinheit 34 aufweist. Beide sind als Spulen ausgeführt, die zueinander fluchtend angeordnet und dazu ausgebildet sind, eine elektrische Leistung drahtlos von dem Versorgungsanschluss 12 zu dem Anwendungsanschluss 10 zu übertragen.

Zwischen den beiden Platinen 26 und 28 ist eine Isolationsstrecke 36 bzw. Funkstrecke 36 vorgesehen, die mit bloßem Auge erkennbar ist und eine Erstreckung von mindestens 0,5 cm aufweist. Die Funkeinheiten 16,16a, 18 und 18a sowie die Kopplungseinheiten 32 und 34 übertragen drahtlos Signale bzw. Daten und eine elektrische Leistung über diese Isolationsstrecke 36, sodass eine galvanische Trennung vorliegt. Die mechanischen Fixierungen 21 sind nicht-leitend ausgeführt. Diese können etwa aus einem Platinenmaterial, aus Kunststoff oder einem anderen nichtleitenden Material hergestellt sein und erlauben lediglich eine günstige Fixierung der Komponenten zueinander.

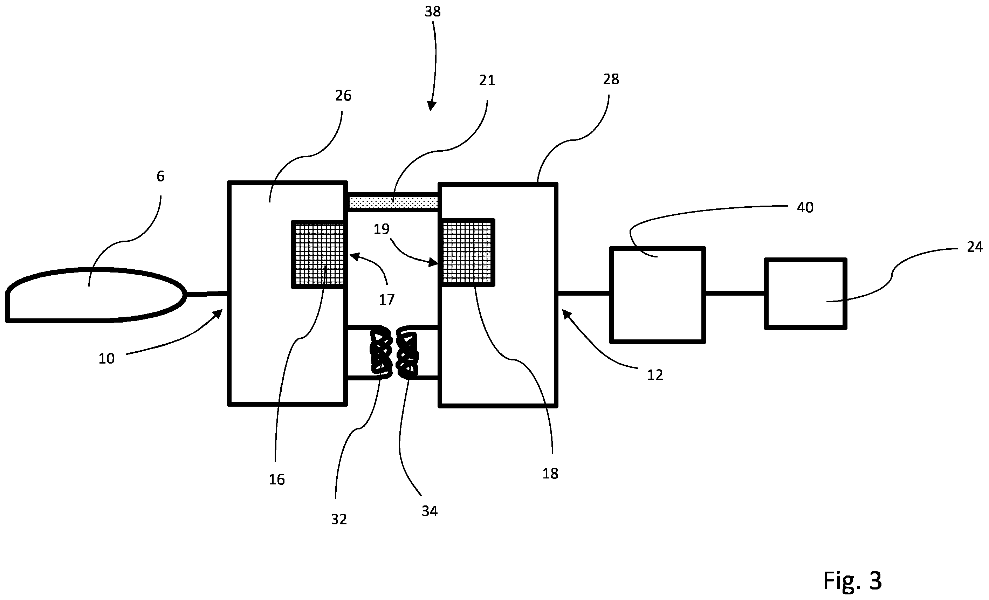

Fig. 3 zeigt eine Abwandlung in Form einer galvanischen Trenneinrichtung 38, bei der lediglich eine einzige erste Funkeinheit 16 und eine einzige zweite Funkeinheit 18 vorgesehen sind. Diese können beispielhaft bidirektional arbeiten und erlauben folglich in einer einfacheren Anordnung eine bidirektionale Kommunikation. Lediglich beispielhaft ist ein Schalter 40 vorgesehen, der mit der Spannungsquelle 24 und dem Versorgungsanschluss 12 verbunden ist.

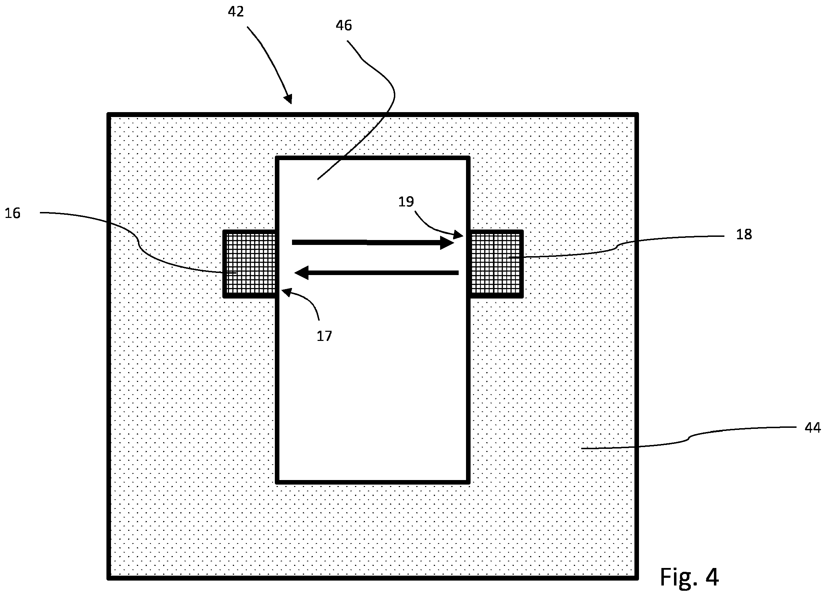

Fig. 4 zeigt schematisch eine galvanische Trenneinrichtung 42, bei der eine erste Funkeinheit 16 und eine zweite Funkeinheit 18 bidirektional kommunizieren können und auf einer einzelnen Platine 44 angeordnet sind. Diese weist einen Ausschnitt 46 über der Isolationsstrecke 36 auf, die optisch wahrnehmbar ist.

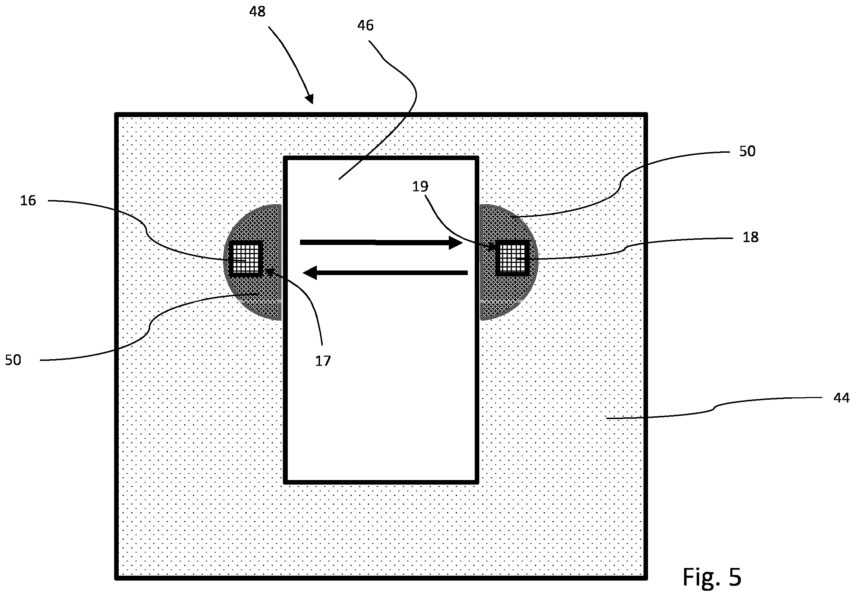

Fig. 5 zeigt einige galvanische Trenneinrichtung 48, die auf der Trenneinrichtung 42 aus Fig. 4 basiert. Hier ist jeweils eine Hornstruktur 50 vorgesehen, die jeweils an der ersten Funkeinheit 16 bzw. der zweiten Funkeinheit 18 angeordnet ist. Beide Hornstrukturen 50 dienen der Ausrichtung der Funkwellen, die von der jeweiligen Funkeinheit 16 bzw. 18 ausgehen. Hierdurch können insbesondere höhere Datenraten bei gleichzeitiger Reduktion der Funkleistung realisiert werden.

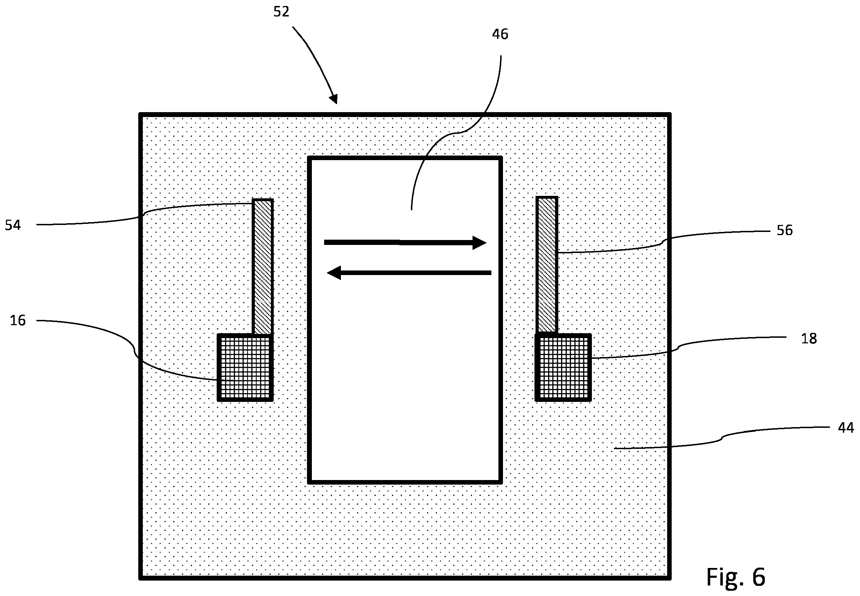

Alternativ dazu könnte eine galvanische Trenneinrichtung 52 gemäß Fig. 6 auch gedruckte Antennen 54 bzw. 56 aufweisen, die randseitig des Zwischenraums 36 angeordnet und zueinander ausgerichtet sind.

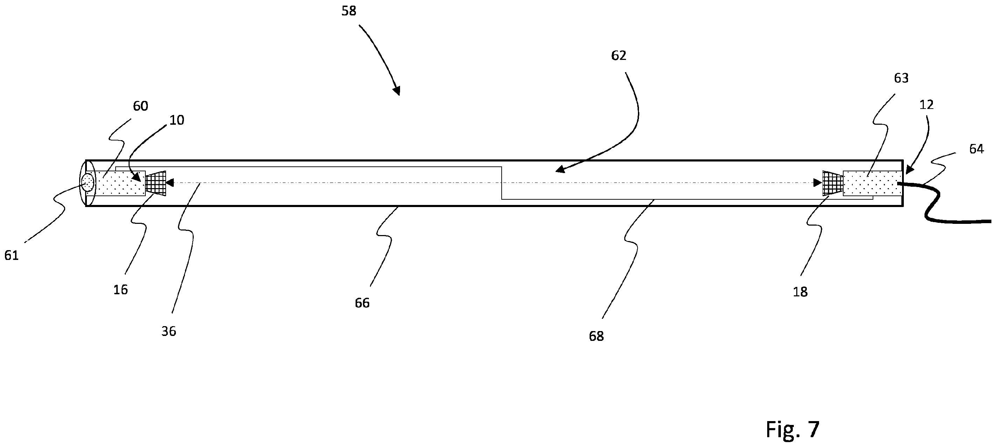

Fig. 7 zeigt eine Anwendungseinrichtung 58 in Form eines Endoskops. Hier ist eine distale Elektronikeinrichtung 60, beispielsweise ein Bildaufnehmer mit einer daran angeordneten Optik 61, in einem distalen Ende angeordnet und über eine galvanische Trenneinrichtung 62 mit einem proximal angeordneten Versorgungsanschluss 12 gekoppelt. Direkt an dem Versorgungsanschluss 12 ist eine proximale Elektronikeinrichtung 63 vorgesehen, die über ein Kabel 64 direkt mit einem Spannungsanschluss oder einer Verarbeitungseinheit verbindbar ist. Die Länge der Anwendungseinrichtung 58 kann groß sein und bis zu etwa 60 cm betragen. Die Isolationsstrecke 36 kann sich in diesem Ausführungsbeispiel über einen wesentlichen Teil dieser Länge erstrecken und folglich bis zu etwa 60 cm betragen. Die Anwendungseinrichtung 58 kann ein Gehäuse 66 umfassen, welches zumindest teilweise aus einem metallischen Material hergestellt ist. Eine Leitung 68 zur Stromversorgung der distalen Elektronikeinheit 60 kann mit einer Kopplungs-einrichtung 30 verbunden sein, die in der proximalen Elektronikeinrichtung 63 oder einer externen Verarbeitungseinheit integriert ist.

### Bezugszeichenliste

- 2: Medizinische Vorrichtung
- 4: Patient
- 6: Anwendungseinrichtung
- 8: Trenneinrichtung
- 10: Anwendungsanschluss
- 12: Versorgungsanschluss
- 14: Einrichtung
- 16, 16a: erste Funkeinheit
- 17, 17a: erste Antenne
- 18, 18a: zweite Funkeinheit
- 19, 19a: zweite Antenne
- 20: Träger
- 21: mechanische Fixierung
- 22: Platine
- 24: Spannungsquelle
- 26: erste Platine
- 28: zweite Platine
- 30: Kopplungseinrichtung
- 32: erste Kopplungseinheit
- 34: zweite Kopplungseinheit
- 36: Isolationsstrecke
- 38: galvanische Trenneinrichtung
- 40: Schalter
- 42: galvanische Trenneinrichtung
- 44: einzelne Platine
- 46: Ausschnitt
- 48: galvanische Trenneinrichtung
- 50: Hornstruktur
- 52: galvanische Trenneinrichtung
- 54: gedruckte erste Antenne
- 56: gedruckte zweite Antenne
- 58: Anwendungseinrichtung
- 60: Elektronikeinrichtung
- 61: Optik
- 62: galvanische Trenneinrichtung
- 63: proximale Elektronikeinrichtung
- 64: Kabel
- 66: Gehäuse
- 68: Leitung

## Patentansprüche

1. Medizinische Vorrichtung (2), aufweisend:
eine mit einem zu behandelnden Patienten (4) in Berührung bringbare Anwendungseinrichtung (6, 58), und
eine galvanische Trenneinrichtung (8, 38, 42, 48, 52, 62), die mit der Anwendungseinrichtung (6, 58) verbindbar ist,
wobei die Trenneinrichtung (8, 38, 42, 48, 52, 62) mindestens einen Anwendungsanschluss (10) zum Verbinden mit der Anwendungseinrichtung (6, 58) und mindestens einen Versorgungsanschluss (12) zum Verbinden mit einer Einrichtung (14) aufweist,
wobei die Trenneinrichtung (8, 38, 42, 48, 52, 62) dazu ausgebildet ist, den Anwendungsanschluss (10) von dem Versorgungsanschluss (12) galvanisch zu trennen,
**dadurch gekennzeichnet, dass** die Trenneinrichtung (8, 38, 42, 48, 52, 62) mindestens eine mit dem Anwendungsanschluss (10) verbundene erste Funkeinheit (16) mit einer ersten Antenne (17, 17a) und mindestens eine mit dem Versorgungsanschluss (12) verbundene zweite Funkeinheit (18) mit einer zweiten Antenne (19, 19a) aufweist,
wobei die jeweilige erste Antenne (17, 17a) und die jeweilige zweite Antenne (19, 19a) auf einem Träger (20, 44) mit bloßem Auge erkennbar voneinander beabstandet und zueinander gerichtet fixiert sind, und
wobei die mindestens eine erste Funkeinheit (16) und die mindestens eine zweite Funkeinheit (18) dazu ausgebildet sind, Signale bzw. Daten zwischen dem Anwendungsanschluss (10) und dem Versorgungsanschluss (12) zu übertragen.

2. Medizinische Vorrichtung (2) nach Anspruch 1, **dadurch gekennzeichnet, dass** die Trenneinrichtung (8, 38, 42, 48, 52, 62) zwei erste Funkeinheiten (16) und zwei zweite Funkeinheiten (18) aufweist, wobei die ersten Funkeinheiten (16) und zweiten Funkeinheiten (18) paarweise einander zugewandt sind, und
wobei eines der Paare zum Übertragen von Signalen bzw. Daten von der ersten Funkeinheit (16) an die zweite Funkeinheit (18) und das andere der Paare zum Übertragen von Signalen bzw. Daten von der zweiten Funkeinheit (18) an die erste Funkeinheit (16) ausgebildet ist.

3. Medizinische Vorrichtung (2) nach Anspruch 1 oder 2, **dadurch gekennzeichnet, dass** die mindestens eine erste Funkeinheit (16) und die mindestens eine zweite Funkeinheit (18) jeweils als Sende- und Empfangseinheit ausgeführt sind.

4. Medizinische Vorrichtung (2) nach einem der vorhergehenden Ansprüche, **dadurch gekennzeichnet, dass** eine zwischen der ersten Antenne (17, 17a) und der zweiten Antenne (19, 19a) vorliegende Funkstrecke (36) eine Ausdehnung von 0,5 bis 60 cm und besonders bevorzugt von 1 bis 40 cm aufweist.

5. Medizinische Vorrichtung (2) nach einem der vorhergehenden Ansprüche, **dadurch gekennzeichnet, dass** der Träger (20, 44) als eine einzelne Platine (44) ausgeführt ist, auf der die mindestens eine erste Funkeinheit (16) und die mindestens eine zweite Funkeinheit (18) in einem vorbestimmten Abstand zueinander montiert sind.

6. Medizinische Vorrichtung (2) nach Anspruch 5, **dadurch gekennzeichnet, dass** die Platine (44) eine Aussparung (46) zwischen der ersten Funkeinheit (16) und der zweiten Funkeinheit (18) aufweist.

7. Medizinische Vorrichtung (2) nach einem der Ansprüche 1 bis 4, **dadurch gekennzeichnet, dass** der Träger (20, 44) eine erste Platine (26) und eine zweite Platine (28) aufweist, die in einem vorbestimmten Abstand zueinander angeordnet sind,
wobei die mindestens eine erste Funkeinheit (16) auf der ersten Platine (26) angeordnet ist und die mindestens eine zweite Funkeinheit (18) auf der zweiten Platine (28) angeordnet ist.

8. Medizinische Vorrichtung (2) nach einem der vorhergehenden Ansprüche, **dadurch gekennzeichnet, dass** die erste Antenne (17, 17a) und die zweite Antenne (19, 19a) auf den Träger (20, 44) gedruckt sind.

9. Medizinische Vorrichtung (2) nach einem der vorhergehenden Ansprüche, **dadurch gekennzeichnet, dass** die erste Antenne (17, 17a) und die zweite Antenne (19, 19a) jeweils eine Hornstruktur (50) zum Herstellen einer gerichteten Funkverbindung aufweist.

10. Medizinische Vorrichtung (2) nach einem der vorhergehenden Ansprüche, **dadurch gekennzeichnet, dass** die Trenneinrichtung (8, 38, 42, 48, 52, 62) eine mit dem Anwendungsanschluss (10) verbundene erste Kopplungseinheit (32) und eine mit dem Versorgungsanschluss (12) verbundene zweite Kopplungseinheit (34) aufweist, die dazu ausgebildet sind, induktiv und/oder kapazitiv elektrische Leistung von dem Versorgungsanschluss (12) an den Anwendungsanschluss (10) zu übertragen.

11. Medizinische Vorrichtung (2) nach einem der vorhergehenden Ansprüche, **dadurch gekennzeichnet, dass** ein Verhältnis v=d/L zwischen einer Distanz (d) zwischen der mindestens einen ersten Funkeinheit (16) und der mindestens einen zweiten Funkeinheit (18) sowie einer Wellenlänge (L) der Signale in einem Bereich von 1,73 bis 127 liegt.

12. Medizinische Vorrichtung (2) nach einem der vorhergehenden Ansprüche, **dadurch gekennzeichnet, dass** ein Bereich zwischen der mindestens einen ersten Funkeinheit (16) und der mindestens einen zweiten Funkeinheit (18) zumindest teilweise mit einem Material ausgefüllt ist, das eine Permittivität von 1 bis 23 F/m und eine Leitfähigkeit von 0 bis 0,01 S/m aufweist.

## Claims

1. A medical apparatus (2) comprising:
an application device (6, 58) which can be brought into contact with a patient (4) to be treated, and
a galvanic isolator (8, 38, 42, 48, 52, 62) which can be connected to the application device (6, 58),
the isolator (8, 38, 42, 48, 52, 62) comprising at least one application connector (10) for connection to the application device (6, 58) and at least one supply connector (12) for connection to a device (14),
the isolator (8, 38, 42, 48, 52, 62) being configured to galvanically isolate the application connector (10) from the supply connector (12),
**characterised in that** the isolator (8, 38, 42, 48, 52, 62) has at least one first radio unit (16), which is connected to the application connector (10) and has a first antenna (17, 17a), and at least one second radio unit (18), which is connected to the supply connector (12) and has a second antenna (19, 19a),
the respective first antenna (17, 17a) and the respective second antenna (19, 19a) being fixed on a carrier (20, 44) at a distance from each other that is visible to the naked eye and being fixed facing each other, and
the at least one first radio unit (16) and the at least one second radio unit (18) being configured to transmit signals and/or data between the application connector (10) and the supply connector (12).

2. The medical apparatus (2) according to claim 1, **characterised in that** the isolator (8, 38, 42, 48, 52, 62) has two first radio units (16) and two second radio units (18),
the first radio units (16) and second radio units (18) facing each other in pairs, and
one of the pairs being configured to transmit signals and/or data from the two first radio unit (16) to the second radio unit (18) and the other of the pairs being configured to transmit signals and/or data from the second radio unit (18) to the first radio unit (16).

3. The medical apparatus (2) according to claim 1 or 2, **characterised in that** the at least one first radio unit (16) and the at least one second radio unit (18) are each embodied as a transmitting and receiving unit.

4. The medical apparatus (2) according to any one of the preceding claims, **characterised in that** a radio path (36) present between the first antenna (17, 17a) and the second antenna (19, 19a) has an extent of from 0.5 to 60 cm and particularly preferably of from 1 to 40 cm.

5. The medical apparatus (2) according to any one of the preceding claims, **characterised in that** the carrier (20, 44) is embodied as a single circuit board (44) on which the at least one first radio unit (16) and the at least one second radio unit (18) are mounted at a predetermined distance from each other.

6. The medical apparatus (2) according to claim 5, **characterised in that** the circuit board (44) comprises a recess (46) between the first radio unit (16) and the second radio unit (18).

7. The medical apparatus (2) according to any one of claims 1 to 4, **characterised in that** the carrier (20, 44) has a first circuit board (26) and a second circuit board (28), which are arranged at a predetermined distance from each other,
the at least one first radio unit (16) being arranged on the first circuit board (26) and the at least one second radio unit (18) being arranged on the second circuit board (28).

8. The medical apparatus (2) according to any one of the preceding claims, **characterised in that** the first antenna (17, 17a) and the second antenna (19, 19a) are printed on the carrier (20, 44).

9. The medical apparatus (2) according to any one of the preceding claims, **characterised in that** the first antenna (17, 17a) and the second antenna (19, 19a) each have a horn structure (50) for establishing a directional radio connection.

10. The medical apparatus (2) according to any one of the preceding claims, **characterised in that** the isolator (8, 38, 42, 48, 52, 62) has a first coupling unit (32) connected to the application connector (10) and a second coupling unit (34) connected to the supply connector (12), which coupling units are configured to inductively and/or capacitively transmit electrical power from the supply connector (12) to the application connector (10).

11. The medical apparatus (2) according to any one of the preceding claims, **characterised in that** a ratio v=d/L between a distance (d) between the at least one first radio unit (16) and the at least one second radio unit (18) and a wavelength (L) of the signals lies in a range of from 1.73 to 127.

12. The medical apparatus (2) according to any one of the preceding claims, **characterised in that** an area between the at least one first radio unit (16) and the at least one second radio unit (18) is at least partially filled with a material that has a permittivity of from 1 to 23 F/m and has a conductivity of from 0 to 0.01 S/m.

## Revendications

1. Dispositif médical (2), présentant :
un dispositif d'application (6, 58) pouvant être amené en contact avec un patient (4) à traiter, et
un dispositif de séparation galvanique (8, 38, 42, 48, 52, 62) qui peut être relié au dispositif d'application (6, 58),
dans lequel le dispositif de séparation (8, 38, 42, 48, 52, 62) présente au moins un raccord d'application (10) pour être relié au dispositif d'application (6, 58) et au moins un raccord d'alimentation (12) pour être relié à un dispositif (14),
dans lequel le dispositif de séparation (8, 38, 42, 48, 52, 62) est conçu pour séparer le raccord d'application (10) du raccord d'alimentation (12) de manière galvanique,
**caractérisé en ce que** le dispositif de séparation (8, 38, 42, 48, 52, 62) présente au moins une première unité radio (16) reliée au dispositif d'application (10) ayant une première antenne (17, 17a) et au moins une deuxième unité radio (18) reliée au dispositif d'alimentation (12) ayant une deuxième antenne (19, 19a),
dans lequel la première antenne (17, 17a) respective et la deuxième antenne (19, 19a) respective sont fixées à distance l'une de l'autre et alignées entre elles de manière visible à l'œil nu sur un support (20, 44),
dans lequel l'au moins une première unité radio (16) et l'au moins une deuxième unité radio (18) sont conçues pour transmettre des signaux, respectivement des données entre le raccord d'application (10) et le raccord d'alimentation (12).

2. Dispositif médical (2) selon la revendication 1, **caractérisé en ce que** le dispositif de séparation (8, 38, 42, 48, 52, 62) présente deux premières unités radio (16) et deux deuxièmes unités radio (18), dans lequel les premières unités radio (16) et les deux unités radio (18) sont tournées l'une vers l'autre par paire, et
dans lequel une des paires est conçue pour transmettre des signaux, respectivement des données de la première unité radio (16) à la deuxième unité radio (18) et l'autre des paires pour transmettre des signaux, respectivement des données de la deuxième unité radio (18) à la première unité radio (16).

3. Dispositif médical (2) selon la revendication 1 ou 2, **caractérisé en ce que** l'au moins une première unité radio (16) et l'au moins une deuxième unité radio (18) sont conçues respectivement en tant qu'unité émettrice et unité réceptrice.

4. Dispositif médical (2) selon l'une des revendications précédentes, **caractérisé en ce qu'**une liaison radio (36) présente entre la première antenne (17, 17a) et la deuxième antenne (19, 19a) présente un allongement de 0,5 à 60 cm et particulièrement de préférence de 1 à 40 cm.

5. Dispositif médical (2) selon l'une des revendications précédentes, **caractérisé en ce que** le support (20, 44) est conçu en tant qu'une platine individuelle (44) sur laquelle l'au moins une première unité radio (16) et l'au moins une deuxième unité radio (18) sont montées à une distance prédéterminée entre elles.

6. Dispositif médical (2) selon la revendication 5, **caractérisé en ce que** la platine (44) présente un évidement (46) entre la première unité radio (16) et la deuxième unité radio (18).

7. Dispositif médical (2) selon l'une des revendications 1 à 4, **caractérisé en ce que** le support (20, 44) présente une première platine (26) et une deuxième platine (28) qui sont disposées à une distance prédéterminée entre elles,
dans lequel l'au moins une première unité radio (16) est disposée sur la première platine (26) et l'au moins une deuxième unité radio (18) est disposée sur la deuxième platine (28).

8. Dispositif médical (2) selon l'une des revendications précédentes, **caractérisé en ce que** la première antenne (17, 17a) et la deuxième antenne (19, 19a) sont pressées sur le support (20, 44).

9. Dispositif médical (2) selon l'une des revendications précédentes, **caractérisé en ce que** la première antenne (17, 17a) et la deuxième antenne (19, 19a) présentent respectivement une structure de corne (50) pour créer une liaison radio dirigée.

10. Dispositif médical (2) selon l'une des revendications précédentes, **caractérisé en ce que** le dispositif de séparation (8, 38, 42, 48, 52, 62) présente une première unité de couplage (32) reliée au raccord d'application (10) et une deuxième unité de couplage (34) reliée au raccord d'alimentation (12), qui sont conçues pour transmettre de la puissance électrique de manière inductive et/ou capacitive du raccord d'alimentation (12) au raccord d'application (10).

11. Dispositif médical (2) selon l'une des revendications précédentes, **caractérisé en ce qu'**un rapport v=d/L entre une distance (d) entre l'au moins une première unité radio (16) et l'au moins une deuxième unité radio (18) ainsi qu'une longueur d'onde (L) des signaux est situé dans une plage de 1,73 à 127.

12. Dispositif médical (2) selon l'une des revendications précédentes, **caractérisé en ce qu'**une partie entre l'au moins une première unité radio (16) et l'au moins une deuxième unité radio (18) est remplie au moins partiellement d'un matériau qui présente une permittivité de 1 à 23 F/m et une conductivité de 0 à 0,01 S/m.
